# EUROPEAN PATENT SPECIFICATION

(11) **EP 1 291 368 B1**
(45) Date of publication and mention of the grant of the patent: **31.05.2017**
(21) Application number: 02718574.3
(22) Date of filing: 12.04.2002
(51) Int. Cl.: A61F 13/531, A61F 13/15, A61L 15/60, C08F 20/06

(54) **WATER-ABSORBING RESIN SUITABLE FOR ABSORBING VISCOUS LIQUID CONTAINING HIGH-MOLECULAR COMPOUND, AND ABSORBENT AND ABSORBENT ARTICLE EACH COMPRISING THE SAME**
WASSERABSORBIERENDES HARZ GEEIGNET FÜR DIE ABSORPTION VISKOSER FLÜSSIGKEITEN ENTHALTEND HOCHMOLEKULARE VERBINDUNGEN SOWIE ABSORBENS UND ABSORBIERENDER GEGENSTAND DIE DIESES ENTHALTEN
RESINE D'ABSORPTION D'EAU APPROPRIEE A L'ABSORPTION DE LIQUIDE VISQUEUX CONTENANT UN COMPOSE A POIDS MOLECULAIRE ELEVE, ET ABSORBANT ET ARTICLE ABSORBANT LES CONTENANT

(30) Priority: 16.04.2001 JP 2001116539
(43) Date of publication of application: 12.03.2003
(73) Proprietor: SUMITOMO SEIKA CHEMICALS CO., LTD., Kako-gun, Hyogo 675-0145 (JP)
(72) Inventor: NAWATA, Yasuhiro, Sumitomo Seiko Chem.Co., Ltd., Himeji-shi, Hyogo 672-8076 (JP); YAMAMORI, Masakazu, Sumitomo Seiko Chem.Co., Ltd., Himeji-shi, Hyogo 672-8076 (JP); UEDA, Koji, Sumitomo Seiko Chem.Co., Ltd., Himeji-shi, Hyogo 672-8076 (JP); YOKOYAMA, Hideki, Sumitomo Seiko Chem.Co., Ltd., Himeji-shi, Hyogo 672-8076 (JP); FUJIKAKE, Masato, Sumitomo Seiko Chem.Co., Ltd., Himeji-shi, Hyogo 672-8076 (JP)
(74) Representative: Boult Wade Tennant
(86) International application number: PCT/JP2002/003706
(87) International publication number: WO 2002/085959

(56) References cited:
- EP-A- 0 317 106
- EP-A- 0 450 924
- EP-A- 0 872 491
- WO-A-95/22357
- WO-A1-97/03114
- JP-A- 04 501 877
- JP-A- 11 060 975
- JP-A- 56 131 608
- US-A- 5 268 224
- US-A- 5 744 564
- US-B1- 6 284 362

## Description

### TECHNICAL FIELD

The present invention relates to a water-absorbent resin suitable for absorbing polymer-containing viscous liquids, and an absorbent core and an absorbent article using the same. Examples of polymer-containing viscous liquids include blood and blood-containing body fluid, as well as watery stool and other types of fecal matter. The water-absorbent resin of the present invention is particularly good at absorbing polymer-containing viscous liquids, and can therefore be successfully used in sanitary napkins, tampons, and other disposable blood-absorbing articles, as well as medical blood-absorbing articles, wound-protecting agents, wound-treating agents, surgical drainage treatment agents, disposable diapers, and other applications.

### BACKGROUND ART

In recent years, water-absorbent resins have come to be widely used in disposable diapers, sanitary products, and other personal hygienic products; water retention agents, soil-conditioning agents, and other agricultural/horticultural materials; cutoff materials, anti-dewing agents, and other industrial materials; and other applications. The use of such resins is particularly widespread in disposable diapers, sanitary products, and other personal hygienic products.

Known examples of water-absorbent resins include hydrolyzed starch/acrylonitrile graft copolymers (JP-B 49-43395), neutralized starch/acrylic acid graft copolymers (JP-A 51-125468), saponified vinyl acetate/acrylic acid ester copolymers (JP-A 52-14689), and partially neutralized polyacrylic acids (JP-A 62-172006, JP-A 57-158209, and JP-A 57-21405).EP 0 872 491 discloses partially neutralized polyacrylic resins made by foam polymerization. Depending on the application, such water-absorbent resins are required to have different absorbent characteristics. Examples of desirable characteristics in the case of personal hygienic applications include (1) high water absorption capacity, (2) high water retention capacity (the amount of water retained by a water-absorbent resin after it has been allowed to absorb water and then dewatered under given conditions), (3) a high water absorption rate, (4) high gel strength following water absorption, and (5) minimal backflow of absorbed liquid to the outside.

Water-absorbent resins used in the field of personal hygienic products are commonly crosslinked to a modest degree. For example, water absorption capacity, post-absorption gel strength, and other water absorption characteristics can be improved to some extent by controlling the degree of crosslinking in the water-absorbent resins used in disposable diapers, incontinence pads, and other products primarily used to absorb human urine.

Water-absorbent resins whose degree of crosslinking is controlled in this manner in accordance with the prior art are disadvantageous, however, in that their absorption capacity, absorption rate, and other parameters of absorption performance decrease dramatically when the absorbed liquid is a polymer-containing viscous liquid such as blood or a blood-containing body fluid, or watery stool or another type of fecal matter. It is not yet clear what the reason is that the absorption performance of a conventional water-absorbent resin decreases dramatically when the absorbed liquid is a polymer-containing viscous liquid. The following tentative explanation can be offered, however.

Polymer-containing viscous liquids have high viscosity and are therefore slow to penetrate between the particles of a water-absorbent resin. Consequently, those particles of the water-absorbent resin that have previously come into contact with a viscous liquid undergo swelling, and the gel thus swelled tends to prevents further passage of liquids. Specifically, gel blocking is apt to occur. It is assumed that viscous liquids are thus impeded in their ability to diffuse between resin particles, making some of the particles that constitute the water-absorbent resin incapable of fully exhibiting their absorption functions.

It is also assumed that when the absorbed liquid is, for example, the watery stool of a newborn or an infant whose staple food is milk, this watery stool is a viscous liquid containing proteins or lipids, so gel blocking is apt to occur due to the deposition of these proteins or lipids on the surfaces of resin particles, with the result that some of the particles constituting the water-absorbent resin can not be used efficiently any more.

When the absorbed liquid is, for example, blood, this blood is a viscous liquid comprising protein-containing plasma components and corporeal components such as erythrocytes, leucocytes, and thrombocytes, so the proteins and corporeal components deposit on the surfaces of the water-absorbent resin particles in a comparatively short time at the start of absorption, enveloping the surfaces of the water-absorbent resin particles. This envelope acts as a barrier and is believed to impede liquids in their ability to penetrate inward from the surfaces of the water-absorbent resin particles.

Several techniques have been proposed with the aim of improving the absorption performance of water-absorbent resins in relation to polymer-containing viscous liquids, and in particular to blood-containing liquids. For example, JP-A 55-505355 discloses a technique in which the surfaces of water-absorbent resin particles are treated with aliphatic hydrocarbons or specific hydrocarbon compounds in order to improve blood dispersion properties. In addition, JP-A 5-508425 discloses a technique in which a specific water-absorbent resin is first coated with an alkylene carbonate and is then heated to 150-300°C in order to improve blood dispersion properties.

In the water-absorbent resins treated in accordance with these conventional techniques, the surfaces of the water-absorbent resin particles have better affinity for blood in the initial period of contact with the blood, but the results are still inadequate in terms of allowing blood to be absorbed all the way into the water-absorbent resin particles, and room for further improvement still remains.

In view of the above, it is an object of the present invention to provide a water-absorbent resin that has an adequate absorption capacity in relation to polymer-containing viscous liquids and that allows polymer-containing viscous liquids such as blood and blood-containing body fluid, as well as watery stool and other types of fecal matter, to disperse between the particles of the water-absorbent resin and to penetrate all the way into the particles of the water-absorbent resin; and to provide an absorbent core and an absorbent article using the same.

As indicated above, the degree of crosslinking in a water-absorbent resin greatly affects the water absorption capacity, water retention capacity, post-absorption gel strength, and other factors.

A low degree of crosslinking tends to provide a water-absorbent resin with a high water absorption capacity because of a loose network structure formed by the crosslinking agent and the polymer chains constituting the water-absorbent resin. However, a low degree of crosslinking tends to reduce the gel strength of the resin because the network structure remains loose after it is swelled and gelled by the liquid absorption, and because the resin has low rubber elasticity.

By contrast, a high degree of crosslinking produces high binding power during water absorption because a dense network structure is created in a water-absorbent resin, with the result that the water absorption capacity tends to decrease. However, such a high degree of crosslinking produces pronounced rubber elasticity because of the dense network structure, with the result that the gel strength tends to increase. For this reason, the resin resists crushing when, for example, placed under a load created by the body. Consequently, the degree of crosslinking must be optimally controlled in accordance with the intended application in the field of personal hygienic products.

The specific surface area of a water-absorbent resin also has a considerable effect on absorption characteristics. A water-absorbent resin is commonly used as a powder composed of spherical, granular, pulverized, or otherwise configured particles. The surface of contact with the absorbed liquid commonly tends to increase and the absorption rate tends to rise with an increase in the specific surface area of the powder.

However, the absorption rate of the absorbed liquid becomes excessively high and the water-absorbent resin undergoes swelling at an early state if an excessively large specific surface area is selected for the water-absorbent resin used in disposable diapers, incontinence pads, and other applications in which the absorbed liquid is human urine. This creates a phenomenon whereby the swelled gel obstructs the flow of liquids, that is gel blocking occurs, and the diffusion properties of the absorbed liquid are adversely affected. For this reason, it becomes difficult for the water-absorbent resin to deliver its inherent performance.

In view of this, the inventors conducted extensive research into the above-mentioned degree of crosslinking and specific surface area in order to obtain a water-absorbent resin capable of adequately absorbing polymer-containing viscous liquids.

As a result, it was discovered that polymer-containing viscous liquids can be absorbed with exceptional efficiency by a water-absorbent resin whose properties are controlled such that the specific surface area of the water-absorbent resin is kept higher and the water retention capacity is kept lower in relation to the level considered optimal for absorbing water or human urine in accordance with the prior art. In particular, the surprising discovery was made that polymer-containing viscous liquids can be absorbed more efficiently by a water-absorbent resin whose water retention capacity is reduced in a controlled manner. The present invention is based on this discovery.

### DISCLOSURE OF THE INVENTION

According to the first aspect of the present invention, a particulate water-absorbent resin suitable for absorbing polymer-containing viscous liquids that are selected from blood, blood-containing body fluid, watery stool and other types of fecal matter,
wherein the resin is a partially neutralized polyacrylic acid,
wherein the resin has an average particle diameter of 50-500 µm, the resin including resin particles having diameters between 180 µm and 355 µm, the resin having a specific surface area of no less than 0.05 m²/g when measured for the resin particles having diameters between 180 µm and 355 µm by a BET multipoint technique using krypton gas as an adsorption gas, as described in the section of the description entitled "(1) Specific Surface Area",
wherein the resin is crosslinked,
wherein the resin has a water retention capacity of 5-30 g/g for 0.9 wt% physiological saline under a centrifugal force of 167 G, as described in the section of the description entitled "(3) Water Retention Capacity",
wherein 0.02 g of the water-absorbent resin exhibits a swelling power of no less than 5 N (newtons) upon lapse of 60 seconds after starting to absorb 0.9 wt% physiological saline, as described in the section of the description entitled "(4) Swelling Power", and
wherein the resin is produced by a process that comprises partially neutralizing an acrylic acid as a monomer in an alkaline aqueous solution, the amount of the monomer in the aqueous solution being between 20 wt% and the saturation concentration; adding a radical polymerization initiator to the aqueous solution containing the partially neutralized monomer; preparing a dispersion medium by adding a surfactant to a petroleum-based hydrocarbon solvent; polymerizing the monomer by reversed-phase suspension polymerization which includes adding to the dispersion medium the aqueous solution containing the partially neutralized monomer and the radical polymerization initiator to prepare a reversed-phase suspension, and heating the thus prepared suspension to form resin particles of the polyacrylic acid; and subjecting the resulting resin particles to surface crosslinking by adding to the suspension a surface crosslinking agent in an amount of from 0.005 mol% to 1 mol% in relation to the total monomer amount.

According to a second aspect of the present invention, an absorbent core suitable for absorbing polymer-containing viscous liquids is provided. The absorbent core is a combination of the above-described water-absorbent resin and a fibrous product.

According to a third aspect of the present invention, there is provided an absorbent article comprising a liquid-permeable sheet, a liquid-impermeable sheet, and an absorbent core disposed therebetween. In this absorbent article, the absorbent core is a combination of the above-described water-absorbent resin and a fibrous product.

### BRIEF DESCRIPTION OF THE DRAWINGS

Fig. 1 is a schematic structural view of an apparatus for measuring the swelling power of the water-absorbent resin of the present invention.
Fig. 2 is a table containing some of the results pertaining to the characteristics of water-absorbent resins in accordance with examples and comparisons.
Fig. 3 is a table with some of the other results pertaining to the characteristics of the water-absorbent resins in accordance with examples and comparisons.

### BEST MODE FOR CARRYING OUT THE INVENTION

The water-absorbent resin of the present invention is produced by reversed-phase suspension polymerization. The water-absorbent resin is obtained by the polymerization of acrylic acid monomers. Such resins are partially neutralized polyacrylic acids.

The water-absorbent resin of the present invention has a specific surface area of 0.05 m²/g or greater, as measured by the BET multipoint technique using krypton gas as the adsorption gas, and a water retention capacity of 5-30 g/g, defined as the ability to retain 0.9 wt% physiological saline. As used herein, "water retention capacity" refers to a value measured by the method described below. Selecting such a structure allows the water-absorbent resin of the present invention to particularly adequately absorb polymer-containing viscous liquids.

The specific surface area of the water-absorbent resin of the present invention, that is, the specific surface area measured by the BET multipoint technique using krypton gas as the adsorption gas is greater than that of a conventional water-absorbent resin commonly used for absorbing water or human urine. Increasing the specific surface area of the water-absorbent resin in this manner makes it possible to increase the absorption rate of the resin in relation to polymer-containing viscous liquids, particularly blood and blood-containing body fluid, as well as watery stool and other types of fecal matter. Increasing the absorption rate increases the liquid absorption rate beyond the rate at which the proteins, corporeal components, and other components contained in such polymer-containing viscous liquids deposit on the surfaces of water-absorbent resin particles and envelop the resin, with the result that these liquids are believed to be facilitated in their ability to penetrate all the way into the water-absorbent resin particles.

It is impossible to ensure an adequate absorption rate in the absorption of a polymer-containing viscous liquid if the specific surface area is less than 0.05 m²/g. The upper limit of the specific surface area is not subject to any particular limitations, but is in practice limited to 5 m²/g or less because of the feasibility limits related to the porosity of the water-absorbent resin particles. The specific surface area should preferably be 0.07-5 m²/g, and should more preferably be 0.10-3 m²/g.

The water retention capacity of the water-absorbent resin of the present invention, that is, the capacity of 1 g of water-absorbent resin to retain 0.9 wt% physiological saline is lower than that of a conventional water-absorbent resin commonly used to absorb water or human urine. The water retention capacity of the water-absorbent resin can be kept within the desired range of numerical values by adjusting the degree of crosslinking. Increasing the degree of crosslinking enhances gel strength in the above-described manner. The water-absorbent resin of the present invention is provided with a higher degree of crosslinking and a greater gel strength than in the past in order to keep the water retention capacity within the aforementioned range. Blocking is less likely to be caused by a swelled gel because the gel strength is higher. As a result, it is believed that viscous liquids can be diffused with greater ease in the water-absorbent resin of the present invention, and a greater number of water-absorbent resin particles can be efficiently utilized.

As such, the inherent water retention capacity of a water-absorbent resin is inadequate if the water retention capacity thereof is less than 5 g/g. Raising the water retention capacity beyond 30 g/g results in poor gel strength and impaired diffusion properties, making it impossible to absorb polymer-containing viscous liquids in an adequate manner. The water retention capacity should more preferably be 10-25 g/g.

Thus, the water-absorbent resin of the present invention is believed to be able to deliver an excellent performance in terms of absorbing polymer-containing viscous liquids as a result of the fact that the absorption rate thereof can be raised by increasing the specific surface area, and the gel strength thereof can be enhanced by increasing the degree of crosslinking.

With the water-absorbent resin of the present invention, the swelling power created when 60 seconds have elapsed after the start of absorption in a case in which 0.02 g of water-absorbent resin is used to absorb 0.9 wt% physiological saline should be 5 N (newtons) or greater, preferably 6.5 N or greater, and more preferably 8 N or greater. The upper limit of the swelling power is not subject to any particular limitations, but in practice about 15 N is established as the limit. As used herein, the term "swelling power" (occasionally referred to as "swelling pressure") refers to the dynamic pressure created in a process in which a water-absorbent resin undergoes swelling, and is expressed in the units (N) of force, as described later. This power can be determined by measuring the force with which a given amount of water-absorbent resin tries to push up a pressure sensor when swelling after the start of absorption.

A close relationship exists between the swelling power and the degree of crosslinking. When the degree of crosslinking is low, an excellent water absorption capacity can be achieved because of the above-described reasons, but the gel strength is low. Consequently, a large number of gels are crushed before the pressure sensor is pushed up, the force that pushes up the pressure sensor decreases, and the swelling power decreases. Conversely, a high degree of crosslinking results in a low water absorption capacity but prevents swelled gels from becoming easily crushed, thereby increasing the force that pushes up the pressure sensor, and enhancing the swelling power.

A water-absorbent resin having a swelling power of 5 N or greater has a high degree of crosslinking and an increased gel strength, making it extremely difficult for gel blocking to occur. Specifically, each gel maintains its strength independently even when the water-absorbent resin particles have gelled, allowing viscous liquids to readily diffuse between the gelled water-absorbent resin particles. It is believed that a viscous liquid dispersed inside aggregated water-absorbent resin particles can readily come into contact with the water-absorbent resin particles disposed further inward, whereby the penetrating viscous liquid can easily reach all the way inside from the surface of the water-absorbent resin, and the resin can deliver an excellent absorption performance.

The water-absorbent resin of the present invention has an average particle diameter of 50-500 µm. It is undesirable for the average particle diameter to be less than 50 µm because in this case the gaps between the water-absorbent resin particles tend to become narrow and gel blocking is apt to occur. Nor is it suitable for the average particle diameter to be greater than 500 µm because such a diameter makes it impossible to obtain an adequate absorption rate.

The method for producing a water-absorbent resin in accordance with the present invention will now be described. The water-absorbent resin of the present invention is produced by reversed-phase suspension polymerization. An example of method used to increase the specific surface area of a water-absorbent resin include a method in which anionic surfactants or nonionic surfactants with an HLB (hydrophilic-lipophilic balance) of 6 or greater are used for performing reversed-phase suspension polymerization. This method will therefore be described below.

In a reversed-phase suspension polymerization method for producing the water-absorbent resin of the present invention, acrylic acid is neutralized in an alkaline aqueous solution as the monomer to be polymerized.

A radical polymerization initiator and, if necessary, a crosslinking agent (referred to hereinbelow as "an internal crosslinking agent"), which is needed to perform crosslinking concurrently with the subsequent polymerization, are then added to the aqueous solution of the neutralization product (aqueous solution of the monomer).

A dispersion medium is subsequently prepared by adding a surfactant to a petroleum-based hydrocarbon solvent and heating and resolving the product, and a reversed-phase suspension is prepared by a process in which the aqueous solution of a neutralization product that contains a radical polymerization initiator and is prepared in the above-described manner is added to the dispersion medium, and the product is stirred. As described above, adding the internal crosslinking agent to this aqueous solution of a neutralization product containing a radical polymerization initiator is optional. In the reversed-phase suspension thus prepared, the aqueous solution of a neutralization product is suspended and dispersed in an oily dispersion medium.

The suspension thus obtained is subsequently heated to a specific polymerization temperature and subjected to a polymerization reaction. As a result, the monomer contained in the suspension undergoes polymerization and forms water-absorbent resin particles.

A specific amount of water is subsequently removed by reheating the suspension, the crosslinking agent (referred to hereinbelow as "the surface crosslinking agent") needed for performing post-polymerization crosslinking is then added, the system is heated to a specific temperature, and a crosslinking reaction is carried out. The oily dispersion medium and water contained in the reaction system are heated and distilled off following the crosslinking reaction.

Excessively small and excessively large particles are finally removed as needed by sieving the water-absorbent resin particles, and the desired water-absorbent resin product is obtained.Acrylic acid is used as a monomer in such a reversed-phase suspension polymerization process .

Aqueous solutions of sodium hydroxide, potassium hydroxide, ammonium hydroxide, and the like can be cited as examples of alkaline aqueous solutions that can be used to neutralize acrylic acids. These alkaline aqueous solutions can be used singly or jointly. Compounds identical to alkali salts obtained by reacting the aforementioned alkaline aqueous solutions with the aforementioned acrylic acids can also be cited as examples of alkali salts of the acrylic acids used when neutralization is dispensed with.

The degree of neutralization provided by an alkaline aqueous solution in relation to all acid groups should preferably range from 10 mol% to 100 mol%, and more preferably from 30 mol% to 80 mol%. It is unsuitable for the degree of neutralization to be less than 10 mol% because of the excessively low water absorption capacity obtained in this case. The pH increases if the degree of neutralization exceeds 100%, so this arrangement is undesirable because of safety considerations.

The monomer concentration of the aqueous monomer solution ranges between 20 wt% and the saturation concentration.

Examples of the radical polymerization initiators added to the aqueous monomer solution include potassium persulfate, ammonium persulfate, sodium persulfate, and other persulfates; methyl ethyl ketone peroxide, methyl isobutyl ketone peroxide, di-*t*-butyl peroxide, *t*-butyl cumyl peroxide, *t*-butyl peroxyacetate, *t*-butyl peroxyisobutyrate, *t*-butyl peroxypivalate, hydrogen peroxide, and other peroxides; and 2,2'-azobis[2-(*N-*phenylamidino)propane]dihydrochloride, 2,2'-azobis[2-(*N-*allylamidino)propane]dihydrochloride, 2,2'-azobis{2-[1-(2-hydroxyethyl)-2-imidazolyn-2-yl]propane}dihydrochloride, 2,2'-azobis{2-methyl-*N*-[1,1-bis(hydroxymethyl)-2-hydroxyethyl]propionamide}, 2,2'-azobis[2-methyl-N-(2-hydroxyethyl)-propionamide], 4,4'-azobis(4-cyanopentanoic acid), and other azo compounds. These radical polymerization initiators can be used singly or as combinations of two or more components.

A radical polymerization initiator is commonly used in an amount of 0.005-1 mol% in relation to the total monomer amount. Using less than 0.005 mol% is undesirable because the subsequent polymerization reaction takes a very long time to complete. Nor is it desirable to use more than 1 mol%, because a violent polymerization reaction takes place in this case.

Redox polymerization may also be carried out by the joint use of sodium sulfite, sodium hydrogensulfite, ferrous sulfate, L-ascorbic acid, and other reducing agents in addition to the aforementioned radical polymerization initiators.

A compound having, for example, two or more polymerizable unsaturated groups may also be used as the internal crosslinking agent arbitrarily added to the aforementioned aqueous monomer solution. Examples of such compounds include di- or tri(meth)acrylic acid esters of (poly)ethylene glycol, (poly)propylene glycol, trimethylol propane, glycerin polyoxyethylene glycol, polyoxypropylene glycol, (poly)glycerin, and other polyols; unsaturated polyesters obtained by reacting the above polyols with maleic acid, fumaric acid, and other unsaturated acids; *N,N-*methylene bis(meth)acrylamide and other bisacrylamides; di-or tri(meth)acrylic acid esters obtained by reacting polyepoxides and (meth)acrylic acid; and di(meth)acrylic acid carbamyl esters obtained by reacting (meth)acrylic acid hydroxyethyl with tolylene diisocyanate, hexamethylene diisocyanate, and other polyisocyanates, as well as allylated starch, allylated cellulose, diallyl phthalate, N,N',N"-triallyl isocyanate, and divinyl benzene.

Compounds having two or more other reactive functional groups may also be used as internal crosslinking agents in addition to the above-mentioned compounds having two or more polymerizable unsaturated groups. Examples of such compounds include (poly)ethylene glycol diglycidyl ether, (poly)propylene glycol diglycidyl ether, (poly)glycerin diglycidyl ether, and other compounds containing glycidyl groups, as well as (poly)ethylene glycol, (poly)propylene glycol, (poly)glycerin, pentaerythritol, ethylenediamine, polyethyleneimine, and glycidyl (meth)acrylate. Two or more such crosslinking agents may be used together. For example, "polyethylene glycol" and "ethylene glycol" will be collectively referred to herein as "(poly)ethylene glycol".

The internal crosslinking agent should be added in an amount of 1 mol% or less, and preferably 0.5 mol% or less, in relation to the total monomer amount. It is unsuitable for the crosslinking agent to be added in an amount greater than 1 mol% because in this case excessive crosslinking develops, and the water-absorbent resin thus obtained has inadequate water absorption properties as a result. The reason that the internal crosslinking agent can be added in an arbitrary manner is that the water retention capacity can be controlled even by the addition of a surface crosslinking agent in order to perform crosslinking on the particle surfaces following monomer polymerization.

Examples of the petroleum-based hydrocarbon solvents used in the preparation of reversed-phase suspensions include *n*-hexane, *n*-heptane, ligroin, and other aliphatic hydrocarbons; cyclopentane, methyl cyclopentane, cyclohexane, methyl cyclohexane, and other alicyclic hydrocarbons; and benzene, toluene, xylene, and other aromatic hydrocarbons. N-Hexane, *n*-heptane, and cyclohexane should preferably be used because they are readily available on a commercial scale, have stable quality, and are inexpensive. These petroleum-based hydrocarbon solvents can be used singly or as combinations of two or more solvents.

Anionic surfactants or nonionic surfactants with an HLB of 6 or greater may be used as the added surfactants. These surfactants can be used singly or as combinations of two or more components.

Examples of such nonionic surfactants include sorbitan fatty acid esters, polyoxyethylene sorbitan fatty acid esters, polyglycerin fatty acid esters, polyoxyethylene glycerin fatty acid esters, sucrose fatty acid esters, sorbitol fatty acid esters, polyoxyethylene sorbitol fatty acid esters, polyoxyethylene alkyl ethers, polyoxyethylene alkyl phenyl ethers, polyoxyethylene castor oil, polyoxyethylene hydrogenated castor oil, alkyl allyl formaldehyde condensate polyoxyethylene ethers, polyoxyethylene polyoxypropylene block copolymers, polyoxyethylene polyoxypropyl alkyl ethers, polyethylene glycol fatty acid esters, alkyl glycosides, N-alkyl glyconamides, polyoxyethylene fatty acid amides, and polyoxyethylene alkylamines.

Examples of anionic surfactants include fatty acid salts, N-acylamino acid salts, polyoxyethylene alkyl ether carboxylates, polyoxyethylene alkyl phenyl ether phosphoric acid ester salts, polyoxyethylene alkyl ether phosphoric acid ester salts, alkylphosphonates, alkylsulfuric acid ester salts, polyoxyethylene alkyl phenyl ether sulfuric acid ester salts, polyoxyethylene alkyl ether sulfuric acid ester salts, higher alcohol sulfuric acid ester salts, polyoxyethylene fatty acid alkanolamide sulfates, alkylbenzenesulfonates, alkylnaphthalenesulfonates, alkylmethyl taurine acid salts, polyoxyethylene alkyl ether sulfonates, and polyoxyethylene alkyl sulfosuccinates.

Among these surfactants, the following are preferred: sorbitan fatty acid esters, polyoxyethylene sorbitan fatty acid esters, polyglycerin fatty acid esters, sucrose fatty acid esters, sorbitol fatty acid esters, polyoxyethylene sorbitol fatty acid esters, polyoxyethylene alkyl phenyl ethers, and other nonionic surfactants.

The surfactants should be used preferably in an amount of 0.1-5 wt%, and more preferably in an amount of 0.2-3 wt%, in relation to the total amount of the aqueous monomer solution. Using the surfactants in an amount of less than 0.1 wt% is unsuitable because in this case the monomers disperse insufficiently, and aggregation is therefore apt to occur during the polymerization reaction. Nor is it suitable to use the surfactants in an amount greater than 5 wt%, because the effect obtained in this case is not commensurate with the amount used, and is therefore uneconomical.

The temperature of the polymerization reaction, while varying with the radical polymerization initiator used, is commonly 20-110°C, and preferably 40-80°C. A reaction temperature below 20°C is economically undesirable because of the low rate of polymerization and extended polymerization time. When the reaction temperature is greater than 110°C, the heat of polymerization is difficult to remove, so it is more difficult to perform the reaction in a smooth manner. When an internal crosslinking agent is added, the crosslinking reaction is performed concurrently because of the heat needed for such polymerization.

Compounds having two or more reactive functional groups can be used as the surface crosslinking agents added following polymerization. Examples thereof include (poly)ethylene glycol diglycidyl ether, (poly)glycerol (poly)glycidyl ether, (poly)propylene glycol diglycidyl ether, (poly)glycerin diglycidyl ether, and other diglycidyl-containing compounds, as well as (poly)ethylene glycol, (poly)propylene glycol, (poly)glycerin, pentaerythritol, ethylenediamine, and polyethyleneimine. Among these, (poly)ethylene glycol diglycidyl ether, (poly)propylene glycol diglycidyl ether, and (poly)glycerin diglycidyl ether are particularly preferred. These crosslinking agents can be used singly or as combinations of two or more agents.

The surface crosslinking agents is added in an amount ranging between 0.005 mol% and 1 mol%, and more preferably between 0.05 mol% and 0.5 mol%, in relation to the total monomer amount. Adding less than 0.005 mol% of a crosslinking agent is unsuitable because the water-absorbent resin obtained in this case has an excessively high water retention capacity. Adding more than 1 mol% of a crosslinking agent is unsuitable because of excessive crosslinking and inadequate water absorption properties.

The surface crosslinking agents should be added in the presence of water preferably in an amount of 0.01-4 weight parts, and more preferably in an amount of 0.05-2 weight parts, per weight part of the water-absorbent resin. The crosslinking occurring near the surfaces of the water-absorbent resin particles can be carried out in a more advantageous manner by controlling the water content during the addition of a surface crosslinking agent in this way.

A hydrophilic organic solvent may also be added as solvent, if necessary, during the addition of the surface crosslinking agent. Examples of such hydrophilic organic solvents include methyl alcohol, ethyl alcohol, *n*-propyl alcohol, isopropyl alcohol, and other lower alcohols; acetone, methyl ethyl ketone, and other ketones; diethyl ether, dioxane, tetrahydrofuran, and other ethers; *N,N-*dimethylformamide and other amides; and dimethyl sulfoxide and other sulfoxides. These hydrophilic organic solvents may be used singly or as combinations of two or more solvents.

The resulting water-absorbent resin of the present invention that is suitable for the absorption of polymer-containing viscous liquids can be shapeless or be shaped as granules, and can be subjected to the various tests described below.

The absorbent core according to the second aspect of the present invention comprises the above-described water-absorbent resin and a fibrous product.

The weight ratio of the water-absorbent resin and fibrous product should preferably range from 1:9 to 9:1, and more preferably range from 3:7 to 7:3.

The following examples of absorbent core structures can be suggested: those in which the water-absorbent resin and the fibrous product are uniformly mixed with each other, and those in which the water-absorbent resin is sandwiched between fibrous products fashioned into sheets or layers. The above two shapes may be combined together, and the structure of the absorbent core is not limited to these shapes alone.

Examples of suitable fibrous products include finely pulverized wood pulp, cotton, cotton linter, rayon, cellulose acetate, and other cellulose-based fibers; and polyamides, polyesters, polyolefins, and other synthetic fibers. Mixtures of the above fibers are also acceptable, and these fibers are not the only possible options.

Individual fibers may be bonded together by adding an adhesive binder in order to enhance the shape retention properties of the absorbent core before or during use. Examples of such adhesive binders include hot-melt synthetic fibers, hot-melt adhesives, and adhesive emulsions.

Examples of such hot-melt synthetic fibers include polyethylene, polypropylene, ethylene-propylene copolymers, and other full-melt binders; and partial-melt binders composed of polypropylene and polyethylene in a side-by-side or core-and-sheath configuration. In the partial-melt binders, the polyethylene portion alone is melted under heating.

Examples of hot-melt adhesives include mixtures of base polymers such as ethylene/vinyl acetate copolymers, styrene/isoprene/styrene block copolymers, styrene/butadiene/ styrene block copolymers, styrene/ethylene/butylene/styrene block copolymers, styrene/ethylene/propylene/styrene block copolymers, and amorphous polypropylene, with tackifiers, plasticizers, antioxidants, and the like.

Examples of adhesive emulsions include polymers of at least one monomer selected from a group consisting of methyl methacrylate, styrene, acrylonitrile, 2-ethylhexyl acrylate, butyl acrylate, butadiene, ethylene, and vinyl acetate. Such adhesive binders can be used singly or as combinations of two or more components.

Sanitary napkins, disposable diapers, and other absorbent articles can be constructed by sandwiching the aforementioned absorbent core between a liquid-permeable sheet and a liquid-impermeable sheet.

Examples of materials that can be used for such liquid-permeable sheets include nonwoven fabrics and porous synthetic resin films composed of polyolefins such as polyethylene and polypropylene, polyesters, polyamides, and the like.

Examples of materials that can be used for such liquid-impermeable sheets include synthetic resin films composed of polyethylene, polypropylene, ethylene vinyl acetate, polyvinyl chloride, and the like; films made of composites of these synthetic resins and nonwoven fabrics; and films made of composites of these synthetic resins with woven products. These liquid-impermeable sheets may also be endowed with vapor-transmitting properties.

The absorbent article thus configured contains a water-absorbent resin suitable for absorbing polymer-containing viscous liquids. The article therefore delivers an excellent performance in terms of absorbing viscous liquids such as menstrual blood and watery stool when used, for example, in sanitary napkins, disposable diapers, and other personal hygienic products. Menstrual blood is prevented from leaking and a so-called dry feel can be obtained when the absorbent article is a sanitary napkin. Leakage can be prevented especially with respect to watery stool and a dry feel can be obtained when the absorbent article is a disposable diaper.

The water-absorbent resin, absorbent core, and absorbent article may further contain amorphous silica, deodorants, antibacterial agents, fragrances, and the like as needed. Various added functions can thereby be obtained.

### <EXAMPLES>

Examples of the present invention will now be described together with comparisons. Following is a description of the test items and test methods adopted for the water-absorbent resins fabricated in the examples and comparisons, and for the absorbent cores fabricated using these resins.

### (1) Specific Surface Area

The water-absorbent resin used in specific surface area measurements was adjusted to a particle diameter that passed through a standard 42-mesh (aperture: 355 µm) JIS (Japanese Industrial Standard) sieve and that was retained at JIS 80 mesh (aperture: 180 µm). The sample was subsequently dried over a period of 16 hours by means of a vacuum drier at a temperature of 100°C and a reduced pressure of about 1 Pa. An adsorption isotherm was then measured at a temperature of 77 K with the aid of a fully automatic precision gas adsorption device (registered trade name: BELSORP36, manufactured by Bel Japan) by a method in which krypton gas was used as the adsorption gas, and specific surface area was determined based on a multipoint BET plot.

### (2) Water Absorption Capacity

1 g of water-absorbent resin was introduced into a teabag (10 x 20 cm) in the form of a pouch made of a woven nylon material with an aperture of 57 µm (255 mesh), and the opening was closed by heat sealing. 1 L of 0.9 wt% physiological saline was then introduced into a beaker with a volume of 1 L, and the teabag was immersed for 1 hour. Following immersion, the teabag was suspended for 10 minutes to remove excess water, and the weight of the entire sample was measured. A teabag devoid of water-absorbent resin was used as a blank whose weight was measured by the same operations. The weight difference between the two was expressed as the amount of water absorption (g), and the numerical value thereof was designated as the water absorption capacity (g/g) of the water-absorbent resin.

### (3) Water Retention Capacity

A teabag subjected to the above-described water retention capacity measurements was introduced into a basket-type centrifugal dehydrator (30 cm in diameter), water was removed therefrom for 60 seconds under conditions corresponding to 1000 rpm (centrifugal force: 167 G), and the overall weight thereof was then measured. A teabag devoid of water-absorbent resin was used as a blank whose weight was measured by the same operations. The weight difference between the two was expressed as the weight (g) of a 0.9 wt% physiological saline retained by the water-absorbent resin, and the numerical value thereof was designated as the water retention capacity (g/g) of the water-absorbent resin.

### (4) Swelling Power

A water-absorbent resin for use in swelling power measurements was adjusted to a particle diameter that passed through a standard 42-mesh (aperture: 355 µm) JIS sieve and that was retained at JIS 80 mesh (aperture: 180 µm).

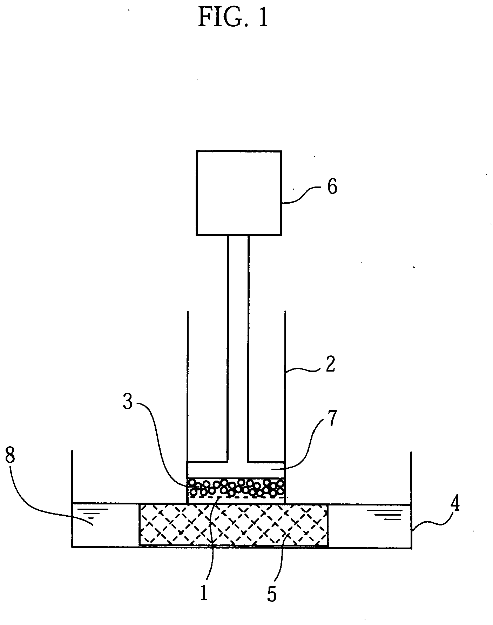

The swelling power of the water-absorbent resin was measured using the apparatus shown in Fig. 1. Specifically, the water-absorbent resin 3 (0.020 g) was introduced at a uniform thickness into an acrylic resin cylinder 2 (bottom surface area: 3.14 cm²) whose inside diameter is 20 mm in which a 255-mesh (aperture: 57 µm) nylon woven fabric 1 was placed on the bottom. A water-permeable glass filter (diameter: 50 mm; thickness: 5 mm) 5 was placed in a laboratory dish 4 with a diameter of 100 mm, and the cylinder 2 was placed on top of the glass filter 5.

Next a pressure sensor 7 whose diameter is 19 mm and which is connected to a load cell 6 was placed immediately above the water-absorbent resin 3 in the cylinder 2 such that no load was applied to the load cell 6. 20 mL of a 0.9 wt% physiological saline 8 was then injected up to a level adjacent to the top surface of the glass filter in the laboratory dish 4. At this point, the physiological saline 8 started to be absorbed by the water-absorbent resin 3 through the glass filter 5 and woven fabric 1. The force exerted by the swelling of the water-absorbent resin 3 that had absorbed the physiological saline 8 was measured by the load cell 6 when 60 seconds had elapsed following the start of absorption, and the numerical value thereof was designated as swelling power (unit: newton (N)).

### (5) Average Particle Diameter

The following standard JIS sieves were sequentially assembled in order from the top down, with a pan at the bottom: 20 mesh (aperture: 850 µm), 32 mesh (aperture: 500 µm), 42 mesh (aperture: 355 µm), 60 mesh (aperture: 250 µm), 80 mesh (aperture: 180 µm), 150 mesh (aperture: 106 µm), and 350 mesh (aperture: 45 µm); a water-absorbent resin (about 100 g) was fed to the topmost sieve; and the assembly was shaken for 20 minutes with the aid of a Ro-Tap shaker.

Next the weight of the water-absorbent resin remaining on each sieve was subsequently calculated as a weight percentage in relation to the total amount. A plurality of calculated values was obtained by sequentially integrating the weight percentages in the direction from smaller particle diameters. The relation between the sieve aperture and the corresponding integrated value was subsequently plotted on logarithmic probability paper. The particle diameter corresponding to an integrated weight percentage of 50% was obtained as the average particle diameter (µm) by connecting the plot on the probability paper by means of a straight line.

### (6) Water Absorption Rate

50 g of a 0.9 wt% physiological saline whose temperature had been adjusted in advance to 25°C was introduced into a beaker with a volume of 100 mL and stirred at a rotational speed of 600 rpm with the aid of a stirring tip (length: 30 mm; diameter: 8 mm). 2 g of a water-absorbent resin was added under stirring. The time between the addition of the resin and the disappearance of vortices on the liquid surface due to the gelation of the water-absorbent resin was measured, and this time was designated as the water absorption rate (seconds).

### (7) Blood Absorption Capacity

0.5 g of water-absorbent resin was introduced into a pouch (10 x 20 cm) made of a 255-mesh woven nylon material (aperture: 57 µm), and the opening was closed by heat sealing. 100 mL of equine blood containing a 3.2% solution of sodium citrate as an anticoagulant in an amount of 10% was introduced into a beaker with a volume of 100 mL, and the sample was immersed in the equine blood for 30 minutes. The hematocrit value of the equine blood was 33%. Following immersion, the sample was suspended for 10 minutes to remove excess blood, and the weight thereof was measured. A nylon pouch devoid of water-absorbent resin was used as a blank whose weight was measured by the same operations. The weight difference between the two was determined, and this value was designated as the blood absorption capacity (g/g) per gram weight of the water-absorbent resin.

### (8) Blood Absorption Properties

1.00 g of water-absorbent resin was uniformly introduced into a laboratory dish with a diameter of 5 cm, and the same type of equine blood (10 g) as that described above was quickly fed dropwise thereto using a pipette. Following the dropwise feeding, the condition of the equine blood absorbed by the water-absorbent resin was visually observed, the time until the absorption of the entire amount was measured, and this time was designated as the first absorption time (seconds). Ten minutes later, another 10 g of equine blood was fed dropwise in the same manner, the time until the absorption of the entire amount was measured, and this time was designated as the second absorption time (seconds).

### (9) Artificial Feces Absorption Properties

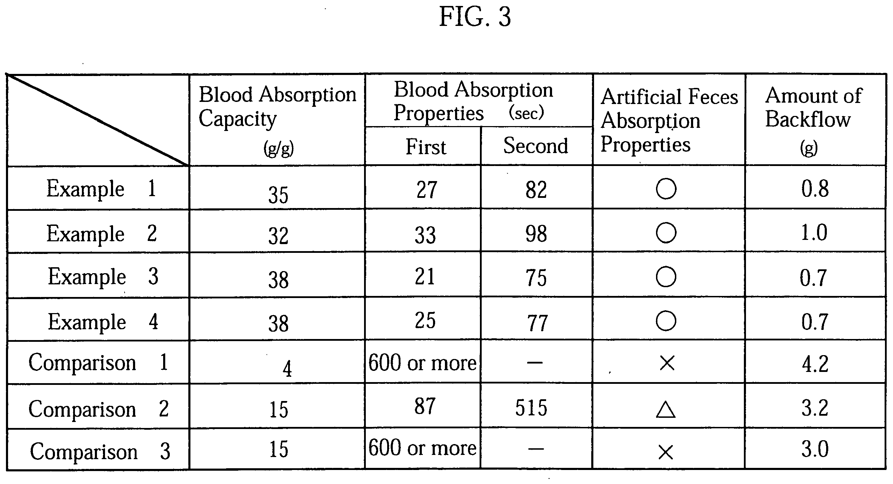

0.6 g of water-absorbent resin was uniformly introduced into a laboratory dish with a diameter of 5 cm, and yogurt with a viscosity of 760 mPa·s (viscosity measurement conditions: B-type viscometer, rotor No. 3, rotational speed 30 rpm) was quickly fed dropwise thereto with a pipette as artificial feces in an amount of 6 g. After the dropwise feeding, the condition of the yogurt absorbed by the water-absorbent resin was visually observed. According to the results of evaluating the absorption properties of artificial feces shown in the table in Fig. 3, circle signs designate cases in which the artificial feces were absorbed substantially completely by the water-absorbent resin, triangle signs designate cases in which a small amount of artificial feces remained on the surface of the water-absorbent resin layer, and multiplication signs designate cases in which hardly any artificial feces were absorbed by the water-absorbent resin.

### (10) Backflow Amount

A sheet-like absorbent core with a size of 5 × 15 cm had a weight of 100 g/m², and comprised a uniform mixture of water-absorbent resin and ground pulp (specific weight 6:4) was fabricated by an air sheet making. Tissue paper was placed on the top and bottom of the absorbent core thus fabricated, the assembly was pressed for 30 seconds under a weight of 98 kPa, and a top sheet made of a polyethylene nonwoven fabric was placed on the top layer, yielding a test absorbent core.

The equine blood (5 mL) described above was dropped near the center of the absorbent core and allowed to stand for 5 minutes. The equine blood (5 mL) was again dropped thereafter and allowed to stand for another 5 minutes. Ten sheets of filter paper (filter paper No. 51A from ADVANTEC) that had been cut to 5 × 15 cm and weighed in advance were then placed near the center, a 5-kg weight (bottom surface size: 5 cm lengthwise and 15 cm sideways) was placed on top, and the weight was kept for 5 minutes. The backflow amount (g) was then determined by measuring the weight of the absorbed equine blood that had flowed back to the filter paper.

### (EXAMPLE 1)

70 g of an 80 wt% aqueous solution of acrylic acid was introduced into a conical flask with a capacity of 500 mL, and the acrylic acid was then neutralized 75 mol% by the dropwise feeding of 111.1 g of a 21 wt% aqueous solution of sodium hydroxide under ice cooling. 0.084 g of potassium persulfate was subsequently added as a radical polymerization initiator to the resulting aqueous solution of partially neutralized acrylic acid.

On the other hand, 550 mL of *n*-Heptane and 0.84 g of sorbitan monolaurate (registered trade name: Nonion LP-20R; HLB value: 8.6; manufactured by NOF Corporation) were added as a petroleum-based hydrocarbon solvent and a surfactant, respectively, to a four-neck cylindrical round-bottom flask with a capacity of 1.5 L that was equipped with a stirrer, a reflux condenser, a dropping funnel, and a nitrogen gas introduction tube, and the system was heated to 50°C. The sorbitan monolaurate was dissolved in the *n*-heptane by heating, and the internal temperature was then reduced to 40°C. The aforementioned aqueous solution of partially neutralized acrylic acid was subsequently added, a reversed-phase suspension was prepared, the interior of the system was replaced with nitrogen gas, and a polymerization reaction was performed for 3 hours at 70°C.

Water was removed from the azeotropic mixture of *n-*heptane and water by reheating the system after the polymerization reaction was completed. 0.2 g of ethylene glycol diglycidyl ether was subsequently added as a surface crosslinking agent, and a crosslinking reaction was carried out. 73.6 g of the water-absorbent resin related to the present invention was obtained by heating and distilling off the *n*-heptane and water from the system after the crosslinking reaction was completed.

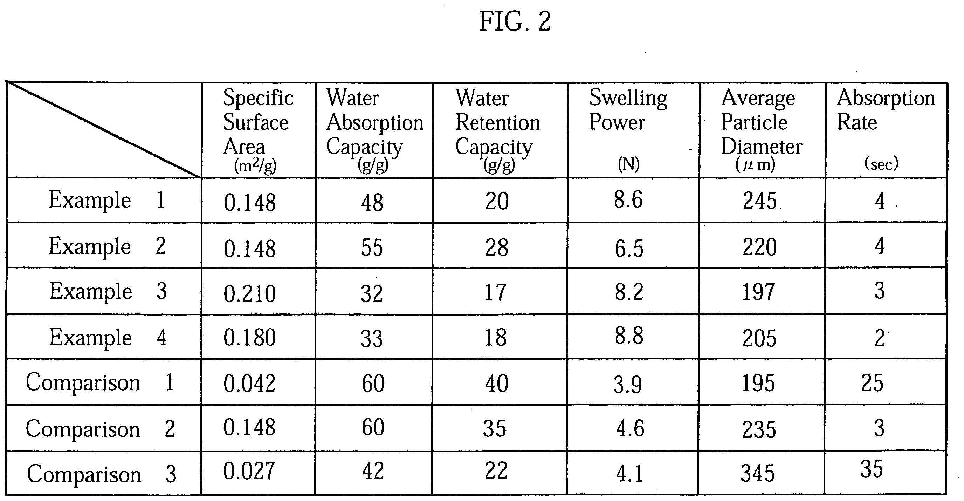

The following parameters of the resulting water-absorbent resin were measured or evaluated by the above-described methods: (1) specific surface area, (2) water absorption capacity, (3) water retention capacity, (4) swelling power, (5) average particle diameter, (6) water absorption rate, (7) blood absorption capacity, (8) blood absorption properties, and (9) artificial feces absorption properties. The above-described absorbent core was fabricated using the resulting water-absorbent resin, and a performance evaluation was conducted for (10) backflow amount. The results are shown in the tables in Figs. 2 and 3.

### (EXAMPLE 2)

72.5 g of water-absorbent resin was produced by the same method as in Example 1 except that the ethylene glycol diglycidyl ether added as a surface crosslinking agent following polymerization was used in an amount of 0.14 g instead of 0.2 g.

The absorption characteristics of the water-absorbent resin pertaining to the present example were measured in the same manner as in Example 1. In addition, an absorbent core was fabricated using this water-absorbent resin in accordance with the above-described method, and the backflow amount was evaluated as a performance characteristic. The results are shown in the tables in Figs. 2 and 3.

### (EXAMPLE 3)

70 g of an 80 wt% aqueous solution of acrylic acid was introduced into a conical flask with a capacity of 500 mL, and the acrylic acid was then neutralized 75 mol% by the dropwise feeding of 111.1 g of a 21 wt% aqueous solution of sodium hydroxide under ice cooling. 0.084 g of potassium persulfate was subsequently added as a radical polymerization initiator to the resulting aqueous solution of partially neutralized acrylic acid.

On the other hand, 550 mL of *n*-Heptane and 1.4 g of sucrose fatty acid ester (registered trade name: Ryoto Sugar Ester S 1170; HLB value: 11; manufactured by Mitsubishi-Kagaku Foods) were added as a petroleum-based hydrocarbon solvent and a surfactant, respectively, to a four-neck cylindrical round-bottom flask with a capacity of 1.5 L that was equipped with a stirrer, a reflux condenser, a dropping funnel, and a nitrogen gas introduction tube, and the system was heated to 50°C. The sucrose fatty acid ester was dissolved in the *n*-heptane by heating, and the internal temperature was then reduced to 40°C. The aforementioned partially neutralized aqueous solution of acrylic acid was subsequently added, a reversed-phase suspension was prepared, the interior of the system was replaced with nitrogen gas, and a polymerization reaction was performed for 3 hours at 70°C.

Water was removed from the azeotropic mixture of *n-*heptane and water by reheating the system after the polymerization reaction was completed. 0.24 g of ethylene glycol diglycidyl ether was subsequently added as a surface crosslinking agent, and a crosslinking reaction was carried out. 73.0 g of the water-absorbent resin related to the present invention was obtained by heating and distilling off the *n*-heptane and water from the system after the crosslinking reaction was completed.

The absorption characteristics of the resulting water-absorbent resin were measured in the same manner as in Example 1. An absorbent core was fabricated using this water-absorbent resin in accordance with the above-described method, and a performance evaluation was conducted for the backflow amount. The results are shown in the tables in Figs. 2 and 3.

### (EXAMPLE 4)

70 g of an 80 wt% aqueous solution of acrylic acid was introduced into a conical flask with a capacity of 500 mL, and the acrylic acid was then neutralized 75 mol% by the dropwise feeding of 111.1 g of a 21 wt% aqueous solution of sodium hydroxide under ice cooling. Potassium persulfate (0.084 g) was subsequently added as a radical polymerization initiator to the resulting aqueous solution of partially neutralized acrylic acid.

On the other hand, 550 mL of *n*-Heptane and 1.4 g of hexaglycerin monostearate (registered trade name: SY-Glyster MS-500; HLB value: 11; manufactured by Sakamoto Yakuhin Kogyo) were added as a petroleum-based hydrocarbon solvent and a surfactant, respectively, to a four-neck cylindrical round-bottom flask with a capacity of 1.5 L that was equipped with a stirrer, a reflux condenser, a dropping funnel, and a nitrogen gas introduction tube, and the system was heated to 50°C. The hexaglycerin monostearate was dissolved in the *n*-heptane by heating, and the internal temperature was then reduced to 40°C. The aforementioned partially neutralized aqueous solution of acrylic acid was subsequently added, a reversed-phase suspension was prepared, the interior of the system was replaced with nitrogen gas, and a polymerization reaction was performed for 3 hours at 70°C.

Water was removed from the azeotropic mixture of *n-*heptane and water by reheating the system after the polymerization reaction was completed. 0.24 g of ethylene glycol diglycidyl ether was subsequently added as a surface crosslinking agent, and a crosslinking reaction was carried out. 73.5 g of the water-absorbent resin related to the present invention was obtained by heating and distilling off the *n*-heptane and water from the system after the crosslinking reaction was completed.

The absorption characteristics of the resulting water-absorbent resin were measured in the same manner as in Example 1. An absorbent core was fabricated using this water-absorbent resin in accordance with the above-described method, and a performance evaluation was conducted for the backflow amount. The results are shown in the tables in Figs. 2 and 3.

### (COMPARISON 1)

92 g of an 80 wt% aqueous solution of acrylic acid was introduced into a conical flask with a capacity of 500 mL, and the acrylic acid was then neutralized 75 mol% by the dropwise feeding of 146.0 g of a 20 wt% aqueous solution of sodium hydroxide under ice cooling. 0.11 g of potassium persulfate was subsequently added as a radical polymerization initiator to the resulting aqueous solution of partially neutralized acrylic acid.

On the other hand, 550 mL of *n*-Heptane and 1.38 g of sucrose fatty acid ester (registered trade name: Ryoto Sugar Ester 370; HLB value: 3; manufactured by Mitsubishi-Kagaku Foods) were added as a petroleum-based hydrocarbon solvent and a surfactant, respectively, to a four-neck cylindrical round-bottom flask with a capacity of 1.5 L that was equipped with a stirrer, a reflux condenser, a dropping funnel, and a nitrogen gas introduction tube, and the system was heated to 50°C. The sucrose fatty acid ester was dissolved in the *n*-heptane by heating, and the internal temperature was then reduced to 40°C. The aforementioned partially neutralized aqueous solution of acrylic acid was subsequently added, the interior of the system was replaced with nitrogen gas, and a polymerization reaction was performed for 3 hours at 70°C.

Water was removed from the azeotropic mixture of *n-*heptane and water by reheating the system after the polymerization reaction was completed. Ethylene glycol diglycidyl ether (0.092 g) was subsequently added as a surface crosslinking agent, and a crosslinking reaction was carried out. A water-absorbent resin (100.5 g) was obtained by heating and distilling off the *n*-heptane and water from the system after the crosslinking reaction was completed.

The absorption characteristics of the resulting water-absorbent resin were measured in the same manner as in Example 1. An absorbent core was fabricated using this water-absorbent resin in accordance with the above-described method, and a performance evaluation was conducted for the backflow amount. The results are shown in the tables in Figs. 2 and 3.

### (COMPARISON 2)

72.0 g of water-absorbent resin was produced by the same method as in Example 1 except that the ethylene glycol diglycidyl ether added as a surface crosslinking agent following polymerization was used in an amount of 0.07 g instead of 0.2 g.

The absorption characteristics of the resulting water-absorbent resin were measured in the same manner as in Example 1. In addition, an absorbent core was fabricated using this water-absorbent resin in accordance with the above-described method, and the backflow amount was evaluated as a performance characteristic. The results are shown in the tables in Figs. 2 and 3.

### (COMPARISON 3)

70 g of an 80 wt% aqueous solution of acrylic acid was introduced into a conical flask with a capacity of 500 mL, and the acrylic acid was then neutralized 75 mol% by the dropwise feeding of 159.4 g of a 14.6 wt% aqueous solution of sodium hydroxide under ice cooling. Next, 0.14 g of *N*,*N'*-methylene bisacrylamide, and 0.07 g of ammonium persulfate and 0.018 g of sodium hydrogensulfite were subsequently added as a crosslinking agent and redox polymerization initiators, respectively, to the resulting aqueous solution of partially neutralized acrylic acid.

Next, the partially neutralized aqueous solution of acrylic acid was subsequently added to a four-neck cylindrical round-bottom flask with a capacity of 1.5 L that was equipped with a stirrer, a reflux condenser, a dropping funnel, and a nitrogen gas introduction tube, the interior of the system was replaced with nitrogen gas, and a polymerization reaction was performed for 3 hours at 70°C. 72.3 g of a water-absorbent resin was obtained by drying and pulverizing the resulting polymer.

The absorption characteristics of the resulting water-absorbent resin were measured in the same manner as in Example 1. An absorbent core was fabricated using this water-absorbent resin in accordance with the above-described method, and a performance evaluation was conducted for the backflow amount. The results are shown in the tables in Figs. 2 and 3.

### [EVALUATION]

An analysis of the tables in Figs. 2 and 3 indicates that the water-absorbent resin of the present invention exhibits excellent absorption properties in relation to polymer-containing viscous liquids. Specifically, it can be seen that a high absorption rate can be established in relation to polymer-containing viscous liquids and that the absorbed liquid, once absorbed, flows back to the outside only minimally even when a load is applied. Consequently, the absorbent core using the water-absorbent resin of the present invention can be successfully used in the field of personal hygienic products, particularly sanitary napkins, tampons, disposable diapers, and other disposable absorbent articles, or in applications such as blood-absorbing articles for medical uses or the like.

## Claims

1. A particulate water-absorbent resin suitable for absorbing polymer-containing viscous liquids that are selected from blood, blood-containing body fluid, watery stool and other types of fecal matter,
wherein the resin is a partially neutralized polyacrylic acid,
wherein the resin has an average particle diameter of 50-500 µm, the resin including resin particles having diameters between 180 µm and 355 µm, the resin having a specific surface area of no less than 0.05 m²/g when measured for the resin particles having diameters between 180 µm and 355 µm by a BET multipoint technique using krypton gas as an adsorption gas, as described in the section of the description entitled "(1) Specific Surface Area",
wherein the resin is crosslinked,
wherein the resin has a water retention capacity of 5-30 g/g for 0.9 wt% physiological saline under a centrifugal force of 167 G, as described in the section of the description entitled "(3) Water Retention Capacity",
wherein 0.02 g of the water-absorbent resin exhibits a swelling power of no less than 5 N (newtons) upon lapse of 60 seconds after starting to absorb 0.9 wt% physiological saline, as described in the section of the description entitled "(4) Swelling Power", and
wherein the resin is produced by a process that comprises partially neutralizing an acrylic acid as a monomer in an alkaline aqueous solution, the amount of the monomer in the aqueous solution being between 20 wt% and the saturation concentration; adding a radical polymerization initiator to the aqueous solution containing the partially neutralized monomer; preparing a dispersion medium by adding a surfactant to a petroleum-based hydrocarbon solvent; polymerizing the monomer by reversed-phase suspension polymerization which includes adding to the dispersion medium the aqueous solution containing the partially neutralized monomer and the radical polymerization initiator to prepare a reversed-phase suspension, and heating the thus prepared suspension to form resin particles of the polyacrylic acid; and subjecting the resulting resin particles to surface crosslinking by adding to the suspension a surface crosslinking agent in an amount of from 0.005 mol% to 1 mol% in relation to the total monomer amount.

2. The particulate water-absorbent resin according to claim 1, wherein 0.02 g of the water-absorbent resin exhibits a swelling power of no more than 15 N (newtons) upon lapse of 60 seconds after starting to absorb 0.9 wt% physiological saline, as described in the section of the description entitled "(4) Swelling Power".

3. An absorbent core comprising a combination of a particulate water-absorbent resin according to claim 1 or 2 and a fibrous product.

4. The absorbent core according to claim 3, wherein the weight ratio of the water-absorbent resin and the fibrous product ranges from 1:9 to 9:1.

5. An absorbent article comprising a liquid-permeable sheet, a liquid-impermeable sheet, and an absorbent core according to claim 3 or 4 disposed therebetween.

6. The absorbent article according to claim 5, which is a sanitary napkin, disposable diaper or other personal hygiene product.

7. Use of the absorbent article according to claim 5 or 6 for absorbing a liquid such as menstrual blood and watery stool.

## Patentansprüche

1. Partikelförmiges wasserabsorbierendes Harz, das zum Absorbieren polymerhaltiger viskoser Flüssigkeiten geeignet ist, die aus Blut, bluthaltigem Körperfluid, wässrigem Stuhl und anderen Arten von Fäkalstoffen ausgewählt sind,
wobei das Harz eine teilweise neutralisierte Polyacrylsäure ist,
wobei das Harz einen mittleren Partikeldurchmesser von 50 bis 500 µm aufweist, wobei das Harz Harzpartikel beinhaltet, die Durchmesser zwischen 180 µm und 355 µm aufweisen, wobei das Harz einen spezifischen Oberflächenbereich von nicht weniger als 0,05 m²/g aufweist, wenn dieser für die Harzpartikel, die Durchmesser zwischen 180 µm und 355 µm aufweisen, durch eine BET-Multipunkttechnik gemessen wird, bei der Kryptongas als Adsorptionsgas verwendet wird, wie dies im Abschnitt der Beschreibung mit dem Titel "(1) Spezifische Oberfläche" beschrieben wird,
wobei das Harz vernetzt ist,
wobei das Harz ein Wasserrückhaltevermögen von 5 bis 30 g/g für eine 0,9 Gew.-%-ige physiologische Kochsalzlösung unter einer Zentrifugalkraft von 167 G aufweist, wie dies im Abschnitt der Beschreibung mit dem Titel "(3) Wasserrückhaltevermögen" beschrieben wird,
wobei 0,02 g des wasserabsorbierenden Harzes nach einem Zeitraum von 60 Sekunden nach dem Beginn der Absorption von 0,9 Gew.-%-iger physiologischer Kochsalzlösung ein Quellvermögen von nicht weniger als 5 N (Newton) aufweisen, wie dies im Abschnitt der Beschreibung mit dem Titel "(4) Quellvermögen" beschrieben wird, und
wobei das Harz durch ein Verfahren hergestellt wird, das umfasst: teilweises Neutralisieren einer Acrylsäure als Monomer in einer alkalischen wässrigen Lösung, wobei die Menge des Monomers in der wässrigen Lösung zwischen 20 Gew.-% und der Sättigungskonzentration beträgt; Zusetzen eines Radikalpolymerisationsinitiators zu der wässrigen Lösung, die das teilweise neutralisierte Monomer enthält; Herstellen eines Dispersionsmediums durch Zusetzen eines Tensids zu einem Kohlenwasserstoff-Lösemittel auf Erdölbasis; Polymerisieren des Monomers durch Umkehrphasen-Suspensionspolymerisation, was das Zusetzen der das teilweise neutralisierte Monomer und den Radikalpolymerisationsinitiator enthaltenden wässrigen Lösung zu dem Dispersionsmedium zur Erzeugung einer Umkehrphasensuspension und das Erhitzen der auf diese Weise hergestellten Suspension zur Bildung von Polyacrylsäure-Harzpartikeln beinhaltet; und Durchführen einer Oberflächenvernetzung mit den resultierenden Harzpartikeln, indem der Suspension ein Oberflächenvernetzungsmittel in einer Menge von 0,005 Mol-% bis 1 Mol-% bezogen auf die gesamte Monomermenge zugesetzt wird.

2. Partikelförmiges wasserabsorbierendes Harz nach Anspruch 1, wobei 0,02 g des wasserabsorbierenden Harzes nach einem Zeitraum von 60 Sekunden nach dem Beginn der Absorption von 0,9 Gew.-%-iger physiologischer Kochsalzlösung ein Quellvermögen von nicht mehr als 15 N (Newton) aufweisen, wie dies im Abschnitt der Beschreibung mit dem Titel "(4) Quellvermögen" beschrieben wird.

3. Absorbierender Kern, umfassend eine Kombination aus einem partikelförmigen wasserabsorbierenden Harz nach Anspruch 1 oder 2 und ein faserförmiges Produkt.

4. Absorbierender Kern nach Anspruch 3, wobei das Gewichtsverhältnis des wasserabsorbierenden Harzes zu dem faserförmigen Produkt im Bereich von 1:9 bis 9:1 liegt.

5. Absorbierender Gegenstand, umfassend eine flüssigkeitsdurchlässige Folie, eine flüssigkeitsundurchlässige Folie und einen dazwischen angeordneten absorbierenden Kern nach Anspruch 3 oder 4.

6. Absorbierender Gegenstand nach Anspruch 5, bei dem es sich um eine Damenbinde, eine Einwegwindel oder ein anderes Produkt zur persönlichen Hygiene handelt.

7. Verwendung des absorbierenden Gegenstands nach Anspruch 5 oder 6 zum Absorbieren einer Flüssigkeit wie Menstruationsblut und wässriger Stuhl.

## Revendications

1. Résine particulaire d'absorption d'eau appropriée à l'absorption de liquides visqueux contenant un polymère qui sont choisis parmi du sang, un fluide corporel contenant du sang, des selles liquides et d'autres types de matières fécales,
dans laquelle la résine est un poly(acide acrylique) partiellement neutralisé,
dans laquelle la résine a un diamètre moyen de particules de 50 à 500 µm, la résine comportant des particules de résine ayant des diamètres compris entre 180 µm et 355 µm, la résine ayant une surface spécifique non inférieure à 0,05 m²/g lorsqu'elle est mesurée pour les particules de résine ayant des diamètres compris entre 180 µm et 355 µm par une technique BET en plusieurs points à l'aide de krypton gazeux en tant que gaz d'adsorption, comme décrit dans la section de la description intitulée « (1) Surface spécifique »,
dans laquelle la résine est réticulée,
dans laquelle la résine a une capacité de rétention d'eau de 5 à 30 g/g pour une solution saline physiologique à 0,9 % en poids sous une force centrifuge de 167 G, comme décrit dans la section de la description intitulée « (3) Capacité de rétention d'eau »,
dans laquelle 0,02 g de résine d'absorption d'eau présente un pouvoir gonflant non inférieur à 5 N (newtons) sur une période de 60 secondes après le début d'une absorption d'une solution saline physiologique à 0,9 % en poids, comme décrit dans la section de la description intitulée « (4) Pouvoir gonflant », et
dans laquelle la résine est produite par un procédé qui comprend la neutralisation partielle d'un acide acrylique en tant que monomère dans une solution aqueuse alcaline, la quantité du monomère dans la solution aqueuse étant comprise entre 20 % en poids et la concentration de saturation ; l'addition d'un initiateur de polymérisation radicalaire à la solution aqueuse contenant le monomère partiellement neutralisé ; la préparation d'un milieu de dispersion par addition d'un tensioactif à un solvant hydrocarboné à base de pétrole ; la polymérisation du monomère par une polymérisation en suspension à phase inversée qui comporte l'addition au milieu de dispersion de la solution aqueuse contenant le monomère partiellement neutralisé et l'initiateur de polymérisation radicalaire pour préparer une suspension à phase inversée, et le chauffage de la suspension ainsi préparée pour former des particules de résine du poly(acide acrylique) ; et la soumission des particules de résine résultantes à une réticulation superficielle par addition à la suspension d'un agent de réticulation superficielle en une quantité de 0,005 % en moles à 1 % en moles par rapport à la quantité totale de monomères.

2. Résine particulaire d'absorption d'eau selon la revendication 1, dans laquelle 0,02 g de résine d'absorption d'eau présente un pouvoir gonflant non supérieur à 15 N (newtons) sur une période de 60 secondes après le début d'une absorption d'une solution saline physiologique à 0,9 % en poids, comme décrit dans la section de la description intitulée « (4) Pouvoir gonflant ».

3. Ame absorbante comprenant une combinaison d'une résine particulaire d'absorption d'eau selon la revendication 1 ou 2 et d'un produit fibreux.

4. Ame absorbante selon la revendication 3, dans laquelle le ratio pondéral de la résine d'absorption d'eau par rapport au produit fibreux s'échelonne de 1:9 à 9:1.

5. Article absorbant comprenant une feuille perméable aux liquides, une feuille imperméable aux liquides et une âme absorbante selon la revendication 3 ou 4 placée entre celles-ci.

6. Article absorbant selon la revendication 5, qui est une serviette hygiénique, une couche jetable ou un autre produit d'hygiène corporelle.

7. Utilisation de l'article absorbant selon la revendication 5 ou 6 pour absorber un liquide tel que le sang menstruel et les selles liquides.
